# EUROPÄISCHE PATENTSCHRIFT

(11) **EP 2 519 216 B1**
(45) Veröffentlichungstag und Bekanntmachung des Hinweises auf die Patenterteilung: **08.02.2017**
(21) Anmeldenummer: 10787138.6
(22) Anmeldetag: 08.12.2010
(51) Int. Cl.: A61K 8/49, A61K 8/58, A61Q 5/04

(54) **HAARBEHANDLUNGSMITTEL UMFASSEND EIN SPEZIELLES TRIALKOXYSILAN UND EINEN ZUSÄTZLICHEN HILFSSTOFF**
HAIR TREATMENT AGENT COMPRISING A SPECIAL TRIALKOXYSILANE AND AN ADDITIONAL AUXILIARY SUBSTANCE
AGENT DE TRAITEMENT CAPILLAIRE COMPRENANT UN TRIALCOXYSILANE SPÉCIAL ET UN ADJUVANT SUPPLÉMENTAIRE

(30) Priorität: 28.12.2009 DE 102009055333
(43) Veröffentlichungstag der Anmeldung: 07.11.2012
(73) Patentinhaber: Henkel AG & Co. KGaA, 40589 Düsseldorf (DE)
(72) Erfinder: EMMERLING, Winfried, 25436 Tornesch (DE); SCHWEINSBERG, Matthias, 22769 Hamburg (DE); ZANDER, Lars, 41569 Rommerskirchen (DE)
(86) Internationale Anmeldenummer: PCT/EP2010/069144
(87) Internationale Veröffentlichungsnummer: WO 2011/080033

(56) Entgegenhaltungen:
- EP-A1- 0 473 039
- EP-A1- 1 736 139
- EP-A1- 1 736 207
- EP-A2- 0 159 628
- FR-A1- 2 926 984
- US-A- 4 344 763

## Beschreibung

Die vorliegende Erfindung betrifft Mittel zur Haarbehandlung, enthaltend mindestens ein spezielles Trialkoxysilan mit mindestens einen zusätzlichen Hilfsstoff, die Verwendung dieser Mittel zur temporären Verformung und/oder zur Pflege keratinhaltiger Fasern und ein entsprechendes Anwendungsverfahren.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Die dauerhafte Haarverformung wird nach den bekannten Dauerwell-Verfahren in der Weise durchgeführt, daß man das Haar mechanisch verformt und die Verformung z. B. durch Aufwickeln auf Haarwickler oder Papilloten festlegt oder unter Anwendung eines Lockeneisens definiert. Vor, während oder nach dieser Verformung behandelt man das Haar mit der wässrigen Zubereitung einer keratinreduzierenden Substanz und spült nach einer Einwirkungszeit mit Wasser oder einer wässrigen Lösung. Manchmal erweist es sich als vorteilhaft, durch Verwendung einer Wärmehaube als Wärmequelle die Umformung zu unterstützen. In einem folgenden Schritt behandelt man dann das Haar mit der wässrigen Zubereitung eines Oxidationsmittels. Nach einer Einwirkungszeit wird auch dieses aus dem Haar ausgespült und das Haar von den mechanischen Verformungshilfsmitteln (Wickler, Papilloten) befreit.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glatt gekämmt. Anstelle des Wicklers ist es auch möglich, die Faser auf ein Glättungsboard glattzulegen. Glättungsboarde sind üblicherweise rechteckige Tafeln z.B. aus Kunststoff. Vorzugsweise ist die Faser dabei mit der keratinreduzierenden Zubereitung benetzt.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bereits bei geringer Einsatzmenge bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Aufgabe der vorliegenden Erfindung war es daher, ein Mittel zur Verformung keratinhaltiger Fasern zur Verfügung zu stellen, das sich durch einen hohen Haltegrad auszeichnet und ein waschstabiles Umformergebnis erzielt.

Ein erster Gegenstand der vorliegenden Erfindung ist daher ein Mittel zur Behandlung keratinhaltiger Fasern, insbesondere menschlichem Haar, gemäß Anspruch 1.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Gemäß allen folgenden Formeln steht eine chemische Bindung, die mit dem Symbol * gekennzeichnet ist, für eine "freie Valenz" des entsprechenden Strukturfragments.

R¹ der Formel (I) steht insbesondere für Methyl oder Ethyl, besonders bevorzugt für Ethyl.

Die Gruppen 3-Aminopropyl, 2-Aminoethyl, Aminomethyl, N,N-3-Dimethylaminopropyl, N,N-2-Dimethylaminoethyl, N,N-Dimethylaminomethyl, N,N-3-Diethylaminopropyl, N,N-2-Diethylaminoethyl oder N,N-Diethylaminomethyl sind bevorzugt geeignete Reste R² der Formel (I). R² der Formel (I) steht bevorzugt für eine Gruppe H₂N-(CH₂)ₙ-* mit n = 1, 2 oder 3 (besonders bevorzugt mit n = 3).

Die Alkoxysilane der Formel (I) des Merkmals (i) und die weiteren Verbindungen des Merkmals (ii) sind verschieden. Die weitere Verbindung des Merkmals (ii) wird unter 1,4-Diazabicyclo[2.2.2]octan (auch als DABCO bezeichnet), 1,5-Diazabicyclo[4,3,0]non-5-en (auch als DBN bezeichnet), 1,8-Diazabicyclo[5,4,0]undec-7-en (aus als DBU bezeichnet) ausgewählt.

Das erfindungsgemäße Mittel enthält das Alkoxysilan der Formel (I) des Merkmals (i) bevorzugt in einer Menge von 0.01 bis 30,00 Gew.-%, besonders bevorzugt von 0,5 bis 20,0 Gew.-%, ganz besonders bevorzugt von 1,0 bis 15 Gew.-%, jeweils bezogen auf das Gewicht des gesamten Mittels.

Das erfindungsgemäße Mittel enthält die weiteren Verbindungen der Merkmals (ii) bevorzugt in einer Menge von 0,001 bis 0,5 Gew.-%, besonders bevorzugt von 0,01 bis 0,1 Gew.-%, jeweils bezogen auf das Gewicht des gesamten anwendungsbereiten Mittels.

Die erfindungsgemäßen Mittel enthalten die Inhalts- bzw- Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Im Rahmen einer bevorzugten Ausführungsform des erfindungsgemäßen Mittel enthält das Mittel daher zusätzlich mindestens einen Alkohol, der 2 bis 6 Kohlenstoffatome und 1 bis 3 Hydroxylgruppen aufweist. Dieser zusätzliche Alkohol wird wiederum bevorzugt ausgewählt aus mindestens einer Verbindung der Gruppe, die gebildet wird aus Ethanol, Ethylenglykol, Isopropanol, 1,2-Propylenglykol, 1,3-Propylenglykol, Glyzerin, n-Butanol, 1,3-Butylenglykol. Ein ganz besonders bevorzugter Alkohol ist Ethanol.

Der zusätzliche Alkohol mit 2 bis 6 Kohlenstoffatomen und 1 bis 3 Hydroxylgruppen ist bevorzugt in dem erfindungsgemäßen Mittel in einer Menge von 20 Gew.-% bis 65 Gew.-%, insbesondere von 25 Gew.-% bis 50 Gew.-%, jeweils bezogen auf das Gewicht des kosmetischen Mittels, enthalten.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel ist Polyethylenglykol in einer Menge bis 30 Gew.-% bezogen auf das gesamte Mittel.

Insbesondere der Zusatz von Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Polyethylenglykol und/oder Polypropylenglykol bezogen auf das gesamte Mittel.

Bevorzugt enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben. Der Einsatz mindestens eines nichtionischen Tensids und/oder mindestens eines kationischen Tensids ist im Rahmen dieser Ausführungsform der Erfindung bevorzugt.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (T-I)

   R¹CO-(OCH₂CHR²)_{w}OR³ (T-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (T-II),

   R⁴O-[G]ₚ (T-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (T-II) gibt den Oligomerisierungsgrad (DP), d. h. die Verteilung von Mono-und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin^{®} CS 15 (COGNIS), Cremophor A25 (BASF SE) bzw. Eumulgin^{®} CS 50 (COGNIS) vermarktet werden.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Die erfindungsgemäßen Mittel können weiterhin die Hilfs- und Zusatzstoffe enthalten, die üblicherweise herkömmlichen Stylingmitteln zugesetzt werden. Als geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton. Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere. Erfindungsgemäß ist es auch möglich, eine Mischung aus mehreren Proteinhydrolysaten einzusetzen.

Die Proteinhydrolysate können in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten sein.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung, enthalten. Mengen von 0,05 - 1 Gew.-% sind besonders bevorzugt.

Vitamin C (Ascorbinsäure). Vitamin C wird in den erfindungsgemäßen Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Grünem Tee, Mandel, Aloe Vera, Kokosnuss, Mango, Aprikose, Limone, Weizen, Kiwi und Melone.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxy-benzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Besonders bevorzugt ist der Einsatz von 4-Methoxyzimtsäure-isopentylester (Isoamyl p-Methoxycinnamate; Neo Heliopan^{®}E 1000).

Bevorzugt sind solche UV-Filter, deren molarer Extinktionskoeffizient am Absorptionsmaximum oberhalb von 15 000, insbesondere oberhalb von 20 000, liegt.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

Weitere Wirk-, Hilfs- und Zusatzstoffe, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate,
- Strukturanten wie Maleinsäure und Milchsäure,
- Parfümöle, Dimethylisosorbid und Cyclodextrine,
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Konsistenzgeber wie Zuckerester, Polyolester oder Polyolalkylether,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Das erfindungsgemäße Mittel wird bevorzugt zur Anwendung auf den keratinhaltigen Fasern aus einer ersten Zusammensetzung, enthaltend in einem kosmetischen Träger mindestens ein Trialkoxysilan der Formel (I) 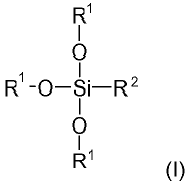 worin
R¹ steht für eine (C₁ bis C₄)-Alkylgruppe,
R² steht für eine Gruppe R'R"N-(CH₂)ₙ-* worin n = 1,2 oder 3, R' und R" unabhängig voneinander für ein Wasserstoffatom oder eine (C₁ bis C₃)-Alkylgruppe stehen,
und einer zweiten Zusammensetzung, enthaltend in einem kosmetischen Träger mindestens eine weitere Verbindung ausgewählt unter den organischen Aminen 1,4-Diazabicyclo[2.2.2]octan, 1,8-Diazabicyclo[5.4.0]undec-7-en und 1,5-Diazabicyclo[4.3.0]non-5-en, gemischt. Die resultierende Mischung ergibt durch die Gegenwart der beiden Komponenten ein reaktives System, welches für den erfindungsgemäßen technischen Effekt verantwortlich ist.

Hierbei sind wiederum die zuvor als bevorzugt genannten Ausführungsformen um die Alkoxysilane der Formel (I) bzw. der weiteren Verbindung *mutatis mutandis* bevorzugt.

Die erste Zusammensetzung enthält als kosmetischen Träger bevorzugt einen alkoholischen kosmetischen Träger, möglich, aber weniger bevorzugt sind wässrigalkoholische Träger. Wenn Wasser in der ersten Zusammensetzung enthalten ist, sollte dessen Menge möglichst klein gehalten werden. Bevorzugt enthält die erste Zusammensetzung weniger als 1.5 Gew.-% freies Wasser. Als Alkohole können insbesondere die für kosmetische Zwecke üblicherweise verwendeten niederen Alkohole mit 1 bis 4 Kohlenstoffatomen wie zum Beispiel Ethanol und Isopropanol benutzt werden.

Im Rahmen einer bevorzugten Ausführungsform enthält die erste Zusammensetzung daher zusätzlich mindestens einen Alkohol, der 2 bis 6 Kohlenstoffatome und 1 bis 3 Hydroxylgruppen aufweist. Dieser zusätzliche Alkohol wird wiederum bevorzugt ausgewählt aus mindestens einer Verbindung der Gruppe, die gebildet wird aus Ethanol, Ethylenglykol, Isopropanol, 1,2-Propylenglykol, 1,3-Propylenglykol, Glyzerin, n-Butanol, 1,3-Butylenglykol. Ein ganz besonders bevorzugter Alkohol ist Ethanol.

Der zusätzliche Alkohol mit 2 bis 6 Kohlenstoffatomen und 1 bis 3 Hydroxylgruppen ist bevorzugt in dem erfindungsgemäßen Mittel in einer Menge von 20 Gew.-% bis 65 Gew.-%, insbesondere von 25 Gew.-% bis 50 Gew.-%, jeweils bezogen auf die gesamte erste Zusammensetzung, enthalten.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel ist Polyethylenglykol in einer Menge bis 30 Gew.-% bezogen auf die gesamte erste Zusammensetzung.

Ein zweiter erfindungsgemäßer Gegenstand ist ein Kit-of-parts gemäß Anspruch 8.

Hierbei sind wiederum die zuvor als bevorzugt genannten Ausführungsformen um die Alkoxysilane der Formel (I) bzw. der weiteren Verbindung *mutatis mutandis* bevorzugt.

Die beiden Zusammensetzungen werden räumlich voneinander getrennt in je einem separaten Container konfektioniert. Bei den jeweiligen Containern kann es sich zum Beispiel um Flaschen oder Dosen beispielsweise aus Materialien wie Kunststoff, Metall oder Glas handeln. Darüber hinaus kann ein Container im Sinne der Erfindung auch eine Kammer eines Mehrkammerbehältnisses sein. Dieses Mehrkammerbehältnis kann eine Vorrichtung umfassen, die es ermöglicht, beide Zusammensetzungen gleichzeitig aus dem Mehrkammerbehältnis zu entnehmen. Hierbei können die Zusammensetzungen vor oder nach dem Austritt aus dem Mehrkammerbehältnis automatisch vermischt werden.

Ein dritter Erfindungsgegenstand ist ein Verfahren zur dauerhaften Umformung keratinhaltiger Fasern, insbesondere menschlicher Haare, in welchem
(i) die Fasern unter Zuhilfenahme von Verformungshilfsmitteln nach, vor oder während Schritt (ii) verformt werden,
(ii) ein Mittel des ersten Erfindungsgegenstandes mit den Fasern in Kontakt gebracht wird,
(iii) die Fasern nach einer Einwirkzeit gegebenenfalls gespült und getrocknet werden.

Verformungshilfsmittel im Sinne des erfindungsgemäßen Verfahrens können
- Wickelhilfen, wie z.B. Lockenwickler oder Papilloten im Falle einer Dauerwelle,
- oder Hilfsmittel für eine mechanische Glättung, wie ein Kamm oder eine Bürste, ein Glättungsboard oder ein beheizbares Glättungseisen im Falle einer Haarglättung sein.

Wenn die Verformungshilfsmittel, beispielsweise Wickelhilfen, im Rahmen eines Wellverfahrens für einen längeren Zeitraum an der Faser befestigt werden, so ist es zweckmäßig, diese Verformungshilfsmittel nach Schritt (iii) zu entfernen.

Die Einwirkzeit beträgt bevorzugt 5 bis 20 Minuten, besonders bevorzugt 10 bis 15 Minuten.

In einer bevorzugten Ausführungsform der Erfindung werden die keratinhaltigen Fasern vor dem Schritt (i) angefeuchtet. Dies kann durch Besprühen der Fasern mit einer Flüssigkeit, beispielsweise mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück. Es ist ferner erfindungsgemäß zum Anfeuchten ein Mittel des ersten oder des zweiten Containers (vgl. Kit-of-parts des zweiten Erfindungsgegenstandes) mit dem Haar in Kontakt zu bringen. Nach der Anwendung von Verformungshilfsmitteln (insbesondere Wicklerhilfen) wird ohne zuvor das Haar zu spülen dasjenige Mittel des erfindungsgemäßen Kit-of-parts auf die Fasern aufgetragen, welches nicht zuvor zum Anfeuchten verwendet wurde. Auf diese Weise wird das erfindungsgemäße Mittel des ersten Erfindungsgegenstandes auf den keratinhaltigen Fasern gebildet. Dann wird weiter gemäß Schritten (ii) und (iii) verfahren.

Die bevorzugt geeigneten Ausführungsformen des erfindungsgemäßen Mittels aus Schritt (ii) sind die im ersten Erfindungsgegenstand genannten (*vide supra*).

Im Rahmen einer weiteren Ausführungsform des erfindungsgemäßen Verfahrens wird das Haar vor Schritt (ii) einer Wärmebehandlung - insbesondere bei Temperaturen von 80°C bis 250°C, besonders bevorzugt bei Temperaturen von 120°C bis 200°C - unterworfen. Als Wärmequelle fungiert dabei bevorzugt ein - insbesondere auf 80°C bis 250°C, besonders bevorzugt auf 120°C bis 200°C - aufgeheizter Feststoff mit dem Haar in direkt Kontakt gebracht wird. Dies ist insbesondere bei der Anwendung von Welleisen oder Glätteisen der Fall.

In einer weiteren Ausgestaltung dieser Ausführungsform werden im Rahmen einer Haarglättung die Fasern im Schritt (iii) einer Wärmebehandlung unter mechanischer Glättung der Faser bei einer Temperatur von 120-220°C unterworfen.

Bei der Anwendung von Welleisen wird eine Haarsträhne um einen entsprechend temperierten gerundeten Körper - z.B einen stabförmigen oder rohrförmigen Körper - gewickelt und nach einer Verweildauer - insbesondere von 10 bis 30 Sekunden - wieder abgewickelt.

Unter einer mechanischen Glättung wird erfindungsgemäß eine Streckung des krausen Haars entlang der längsten räumlichen Ausdehnung der Haarfaser verstanden.

Die Wärmebehandlung unter mechanischer Glättung des Haars findet bevorzugt bei einer Temperatur von 140-200°C statt. Die Wärmebehandlung kann mit heißer Luft erfolgen. In diesem Fall, wird das Haar während des Kämmens genau an der Stelle erwärmt, an der die mechanische Glättung erfolgt.

Darüber hinaus ist es besonders bevorzugt, dass die Wärmebehandlung nach Manier des Glättens mit Hilfe entsprechend temperierter Platten, insbesondere Metall- oder Keramikplatten, erfolgt, in dem die Platte auf die zu glättenden Haare gedrückt wird und die an das Haar gedrückte Platte entlang der Haarfaser bewegt wird. Die Platten können gegebenenfalls mit hitzebeständigen Werkstoffen beschichtet sein. Besonders bevorzugt wird die zu glättende Haarfaser zwischen zwei entsprechend temperierte Platten gepresst und beide Platten zugleich entlang der längsten räumlichen Ausdehnung der Faser bewegt. Dabei ist es wiederum bevorzugt, dass beide Platten miteinander verbunden sind, so dass beide Platten gleichmäßig entlang der Haarfaser bewegt werden können. Wird die Wärmebehandlung am lebenden Haar durchgeführt, so ist die Haarfaser an einem Ende (Haarwurzel) befestigt. Die Platten werden in diesem Fall bevorzugt gleichmäßig von der Haarwurzel weg entlang der gesamten Haarfaser bewegt. Durch diese Bewegung erfolgt eine mechanische Glättung der Faser. Ein entsprechendes Gerät zur Wärmebehandlung ist beispielsweise das Gerät "Ceramic Flat-Master" (Vertrieben durch: Efalock, Deutschland).

Die nachfolgenden Beispiele sollen den Gegenstand der vorliegenden Erfindung erläutern ohne ihn in irgendeiner Weise zu beschränken.

### Beispiele

Die folgenden Mengenangaben verstehen sich - soweit nichts anderes vermerkt ist - in Gewichtsprozent.

Folgende Rezepturen wurden durch Vermischen der angegebenen Rohstoffe bereitgestellt:

| **Rohstoff** | **E1A** | **E1B** | **V1** |
|---|---|---|---|
| 3-Aminopropyltriethoxysilan | 10,0 | - | 5,0 |
| Diazabicylooctan (DABCO) | - | 0,5 | - |
| Ethanol | 90,0 | - | 45,0 |
| Wasser | - | 99,5 | 50,0 |

Komponente E1A und E1B wurden kurz vor der Anwendung im Gewichtsverhältnis von 1 zu 1 gemischt.

Haarsträhnen Alkinco 6634, natural dark European hair (Fa. Alkinco) wurden gewaschen und auf Spiralwickler aufgewickelt. Auf eine Haarsträhne wurde pro g Haar 1 ml der erfindungsgemäßen Anwendungsmischung bzw. auf eine andere Haarsträhne das frisch zubereitete Mittel V1 entsprechend aufgetragen und 15 Minuten lang einwirken gelassen. Anschließend wurde jede Strähne je 5 Minuten mit Wasser gespült und an der Luft trocknen gelassen. Die Spiralwickler wurden entfernt und die Strähnen 24 h in eine Klimakammer mit 90% rel. Luftfeuchtigkeit gehängt.

Die Strähnen, welche mit dem erfindungsgemäßen Mittel behandelt wurden waren nach 24h Lagerung bei 90% rel. Luftfeuchtigkeit deutlich formstabiler.

Um die Waschstabilität des obigen Umformungsergebnisses zu prüfen, wurden die besagten Strähnen in einem weiteren Schritt mit einem handelsüblichen Haarshampoo gewaschen und an der Luft getrocknet. Wiederum wurden die Strähnen für 24 h in eine Klimakammer mit 90% rel. Luftfeuchtigkeit gehängt.

Die Strähnen, die mit dem erfindungsgemäßen Mittel behandelt wurden, zeigten deutlich ausgeprägtere Locken als die Vergleichssträhnen.

## Patentansprüche

1. Mittel zur Behandlung keratinhaltiger Fasern, insbesondere menschlichem Haar, enthaltend in einem kosmetisch akzeptablen Träger
(i) mindestens ein Trialkoxysilan der Formel (I) 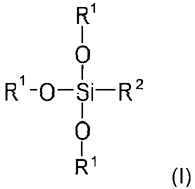 worin
R¹ steht für eine (C₁ bis C₄)-Alkylgruppe,
R² steht für eine Gruppe R'R"N-(CH₂)ₙ-* worin n = 1,2 oder 3, R' und R" unabhängig voneinander für ein Wasserstoffatom oder eine (C₁ bis C₃)-Alkylgruppe stehen,
und
(ii) mindestens eine weitere Verbindung, ausgewählt unter den organischen Aminen 1,4-Diazabicyclo[2.2.2]octan, 1,8-Diazabicyclo[5,4,0]undec-7-en und 1,5-Diazabicyclo[4,3,0]non-5-en.

2. Mittel nach Anspruch 1, **dadurch gekennzeichnet, dass** R¹ der Formel (I) steht für Methyl oder Ethyl, besonders bevorzugt für Ethyl.

3. Mittel nach einem der vorangehenden Ansprüche, **dadurch gekennzeichnet, dass** der Rest R² der Formel (I) für eine Gruppe H₂N-(CH₂)ₙ-* mit n = 1, 2 oder 3, insbesondere mit n = 3, steht.

4. Mittel nach einem der Ansprüche 1 oder 2, **dadurch gekennzeichnet, dass** der Rest R² gemäß Formel (I) für eine Gruppe 3-Aminopropyl, 2-Aminoethyl, Aminomethyl, N,N-3-Dimethylaminopropyl, N,N-2-Dimethylaminoethyl, N,N-Dimethylaminomethyl, N,N-3-Diethylaminopropyl, N,N-2-Diethylaminoethyl oder N,N-Diethylaminomethyl steht.

5. Mittel nach einem der vorangehenden Ansprüche, **dadurch gekennzeichnet, dass** das Alkoxysilan der Formel (I) in einer Menge von 0.01 bis 30,00 Gew.-%, bezogen auf das Gewicht des gesamten Mittels, enthalten ist.

6. Mittel nach einem der vorangehenden Ansprüche, **dadurch gekennzeichnet, dass** die weiteren Verbindungen der Merkmals (ii) in einer Menge von 0,001 bis 0,5 Gew.-%, besonders bevorzugt von 0,01 bis 0,1 Gew.-%, jeweils bezogen auf das Gewicht des gesamten anwendungsbereiten Mittels, enthalten sind.

7. Mittel nach einem der vorangehenden Ansprüche, **dadurch gekennzeichnet, dass** es zusätzlich mindestens einen Alkohol, der 2 bis 6 Kohlenstoffatome und 1 bis 3 Hydroxylgruppen aufweist.

8. Kit-of-parts umfassend
- in einem ersten Container eine Zusammensetzung, enthaltend in einem kosmetischen Träger mindestens ein Trialkoxysilan der Formel (I) 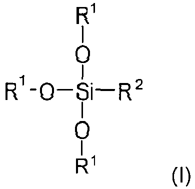 worin
R¹ steht für eine (C₁ bis C₄)-Alkylgruppe,
R² steht für eine Gruppe R'R"N-(CH₂)ₙ-* worin n = 1,2 oder 3, R' und R" unabhängig voneinander für ein Wasserstoffatom oder eine (C₁ bis C₃)-Alkylgruppe stehen,
und
- in einem zweiten Cotainer eine Zusammensetzung, enthaltend in einem kosmetischen Träger mindestens eine weitere, Verbindung ausgewählt unter den organischen Aminen 1,4-Diazabicyclo[2.2.2]octan, 1,8-Diazabicyclo[5,4,0]undec-7-en und 1,5-Diazabicyclo[4,3,0]non-5-en.

9. Verfahren zur dauerhaften Umformung keratinhaltiger Fasern, insbesondere menschlicher Haare, in welchem
(i) die Fasern unter Zuhilfenahme von Verformungshilfsmitteln nach, vor oder während Schritt (ii) verformt werden,
(ii) ein Mittel der Ansprüche 1 bis 7 mit den Fasern in Kontakt gebracht wird,
(iii) die Fasern nach einer Einwirkzeit gegebenenfalls gespült und getrocknet werden.

10. Verfahren nach Anspruch 9, **dadurch gekennzeichnet, dass** die Einwirkzeit 5 bis 20 Minuten, bevorzugt 10 bis 15 Minuten, beträgt.

11. Verfahren nach einem der Ansprüche 9 oder 10, **dadurch gekennzeichnet, dass** im Rahmen einer Haarglättung die Fasern im Schritt (iii) einer Wärmebehandlung unter mechanischer Glättung der Faser bei einer Temperatur von 120-220°C unterworfen werden.

## Claims

1. An agent for treating keratin-containing fibers, in particular human hair, containing, in a cosmetically acceptable carrier,
(i) at least one trialkoxysilane of formula (I) 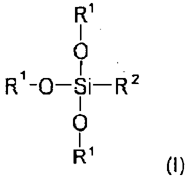 where
R¹ represents a (C₁ to C₄) alkyl group,
R² represents a group R'R"N-(CH₂)ₙ-* where n = 1, 2 or 3 and R' and R" independently of one another represent a hydrogen atom or a (C₁ to C₃) alkyl group,
and
(ii) at least one additional compound, selected from the organic amines: 1,4-diazabicyclo[2.2.2]octane, 1,8-diazabicyclo[5,4,0]undec-7-ene and 1,5-diazabicyclo[4,3,0]non-5-ene.

2. The agent according to claim 1, **characterized in that** R¹ of formula (I) represents methyl or ethyl, more preferably ethyl.

3. The agent according to one of the preceding claims, **characterized in that** the functional group R² of formula (I) represents a group H₂N-(CH₂)ₙ-*, where n = 1, 2 or 3, in particular n = 3.

4. The agent according to one of claims 1 or 2, **characterized in that** the functional group R² according to formula (I) represents a group selected from 3-aminopropyl, 2-aminoethyl, aminomethyl, N,N-3-dimethylaminopropyl, N,N-2-dimethylaminoethyl, N,N-dimethylaminomethyl, N,N-3-diethylaminopropyl, N,N-2-diethylaminoethyl or N,N-diethylaminomethyl.

5. The agent according to one of the preceding claims, **characterized in that** said agent contains the alkoxysilane of formula (I) in an amount of from 0.01 to 30.00 wt.%, based on the weight of the total agent.

6. The agent according to one of the preceding claims, **characterized in that** said agent contains the additional compounds of feature (ii) in an amount of from 0.001 to 0.5 wt.%, more preferably from 0.01 to 0.1 wt.%, in each case based on the weight of the total ready-to-use agent.

7. The agent according to one of the preceding claims, **characterized in that** said agent additionally contains at least one alcohol which comprises from 2 to 6 carbon atoms and from 1 to 3 hydroxyl groups.

8. A kit of parts, comprising
- in a first container a composition that contains in a cosmetic carrier at least one trialkoxysilane of formula (I) 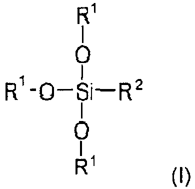 where
R¹ represents a (C₁ to C₄) alkyl group,
R² represents a group R'R"N-(CH₂)ₙ-*, where n = 1, 2 or 3 and R' and R" independently of one another represent a hydrogen atom or a (C₁ to C₃) alkyl group,
and
- comprising in a second container a composition that contains in a cosmetic carrier at least one additional compound selected from the organic amines 1,4-diazabicyclo[2.2.2]octane, 1,8-diazabicyclo[5.4.0]undec-7-ene and 1,5-diazabicyclo[4.3.0]non-5-ene.

9. A method for the long-lasting shaping of keratin-containing fibers, in particular human hair, in which method
(i) the fibers are shaped with the aid of shaping means after, before or during step (ii),
(ii) an agent according to claims 1 to 7 is brought into contact with the fibers,
(iii) the fibers are optionally rinsed and dried after an application time.

10. The method according to claim 9, **characterized in that** the application time is from 5 to 20 minutes, preferably from 10 to 15 minutes.

11. The method according to one of claims 9 or 10, **characterized in that**, in the context of hair straightening, in step (iii) the fibers undergo thermal treatment by means of mechanical straightening of the fibers at a temperature of from 120-220 °C.

## Revendications

1. Produit de traitement de fibres contenant de la kératine, en particulier du cheveu humain, contenant dans un vecteur cosmétiquement acceptable :
i) au moins un trialcoxysilane de formule (I) 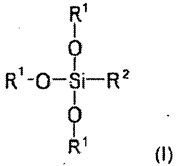 où
R¹ représente un groupe alkyle en C₁ à C₄,
R² représente un groupe R'R"N-(CH₂)ₙ-* avec n = 1, 2 ou 3, et R' et R" représentant indépendamment l'un de l'autre un atome d'hydrogène ou un groupe alkyle en C₁ à C₃,
et
ii) au moins un autre composé choisi parmi les amines organiques 1,4-diazabicyclo[2.2.2]octane, 1,8-diazabicyclo[5.4.0]undécan-7-ène et 1,5-diazabicyclo[4.3.0]non-5-ène.

2. Produit selon la revendication 1, **caractérisé en ce que** R¹ dans la formule (I) représente méthyle ou éthyle, particulièrement préférentiellement éthyle.

3. Produit selon une des revendications précédentes, **caractérisé en ce que** le résidu R² dans la formule (I) représente un groupe H₂N-(CH₂)ₙ-* avec n = 1, 2 ou 3, en particulier 3.

4. Produit selon la revendication 1 ou 2, **caractérisé en ce que** le résidu R² dans la formule (I) représente un groupe 3-aminopropyle, 2-aminoéthyle, aminométhyle, N,N-3-diméthylaminopropyle, N,N-2-diméthylaminoéthyle, N,N-diméthylaminométhyle, N,N-3-diéthylaminopropyle, N,N-2-diéthylaminoéthyle ou N,N-diéthylaminométhyle.

5. Produit selon une des revendications précédentes, **caractérisé en ce que** l'alcoxysilane dans la formule (I) est présent à hauteur de 0,01 à 30,00 % en poids rapporté au poids du produit total.

6. Produit selon une des revendications précédentes, **caractérisé en ce que** les autres composés de la caractéristique ii) sont présents à hauteur de 0,001 à 0,5 % en poids, particulièrement préférentiellement de 0,01 à 0,1 % en poids rapporté au poids du produit total du produit total prêt à l'emploi.

7. Produit selon une des revendications précédentes, **caractérisé en ce qu'**il présente en plus au moins un alcool avec 2 à 6 atomes de carbone et 1 à 3 groupes hydroxyles.

8. Kit d'éléments comprenant :
- dans un premier récipient, une composition contenant, dans un vecteur cosmétiquement acceptable, au moins un trialcoxysilane de formule (I) 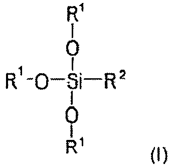 où
R¹ représente un groupe alkyle en C₁ à C₄,
R² représente un groupe R'R"N-(CH₂)ₙ-* avec n = 1, 2 ou 3, et R' et R" représentant indépendamment l'un de l'autre un atome d'hydrogène ou un groupe alkyle en C₁ à C₃,
et
- dans un deuxième récipient, une composition contenant, dans un vecteur cosmétiquement acceptable, au moins un autre composé choisi parmi les amines organiques 1,4-diazabicyclo[2.2.2]octane, 1,8-diazabicyclo[5.4.0]undécan-7-ène et 1,5-diazabicyclo[4.3.0]non-5-ène.

9. Procédé de déformation durable de fibres contenant de la kératine, en particulier du cheveu humain, dans lequel :
i) on déforme les fibres à l'aide de produits d'aide à la déformation après, avant ou pendant l'étape ii),
ii) on met en contact un produit selon les revendications 1 à 7 avec les fibres,
iii) éventuellement, on rince et on sèche les fibres à l'issue d'une durée d'application.

10. Procédé selon la revendication 9, **caractérisé en ce que** la durée d'application est de 5 à 20 minutes, de préférence de 10 à 15 minutes.

11. Procédé selon une des revendications 9 ou 10 **caractérisé en ce que**, dans le cadre d'un lissage capillaire, les fibres sont soumises à l'étape iii) à un traitement thermique avec lissage mécanique des fibres à une température de 120 à 220°C.
